(19) 

Europäisches
Patentamt
European
Patent Office
Office européen
des brevets

(11) **EP 4 166 156 A1**

(12) **EUROPEAN PATENT APPLICATION**
published in accordance with Art. 153(4) EPC

(43) Date of publication:
**19.04.2023 Bulletin 2023/16**

(21) Application number: **21822836.9**

(22) Date of filing: **11.06.2021**

(51) International Patent Classification (IPC):
*A61K 38/08* (2019.01)          *A61P 37/08* (2006.01)
*A61P 11/06* (2006.01)          *A61P 27/14* (2006.01)
*A61P 17/00* (2006.01)          *A61K 8/64* (2006.01)
*A61Q 19/00* (2006.01)

(52) Cooperative Patent Classification (CPC):
**A61K 8/64; A61K 38/08; A61P 11/06; A61P 17/00;**
**A61P 27/14; A61P 37/08; A61Q 19/00**

(86) International application number:
**PCT/KR2021/007322**

(87) International publication number:
**WO 2021/251788 (16.12.2021 Gazette 2021/50)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **12.06.2020 KR 20200071658**

(71) Applicant: **Caregen Co., Ltd.**
**Anyang-si, Gyeonggi-do 14119 (KR)**

(72) Inventors:
• **CHUNG, Yong Ji**
**Anyang-si, Gyeonggi-do 14119 (KR)**

• **KIM, Eun Mi**
**Anyang-si, Gyeonggi-do 14119 (KR)**
• **KIM, Seon Soo**
**Anyang-si, Gyeonggi-do 14119 (KR)**

(74) Representative: **Regimbeau**
**20, rue de Chazelles**
**75847 Paris Cedex 17 (FR)**

Remarks:
The complete document including Reference
Table(s) and the Sequence Listing(s) can be
downloaded from the EPO website

(54) **COMPOSITION FOR PREVENTING, AMELIORATING, OR TREATING ALLERGIC DISEASES OR PRURITUS, CONTAINING PENTAPEPTIDE AS ACTIVE INGREDIENT**

(57) The present invention related to a composition for the prevention, improvement or treatment of allergic diseases or itching.

【Figure 3】

**Description**

[Technical Field]

The present invention relates to use of pentapeptide for the prevention, improvement or treatment of allergic diseases or itching.

[Background Art]

Mast cells and blood basophils are known as cells in the body that induce various allergic diseases by secreting cytokines. These cells have a receptor (FcεRI) against IgE, an antibody that causes allergy on the cell surface, and it is stimulated by an allergy-causing substance (called an antigen or allergen) to secrete its various allergy-causing substances out of the cell (Amin K., Respiratory Medicine., 2012).

Atopic Dermatitis (AD), one of the allergic diseases, is a chronic inflammatory skin disease that causes dryness, pruritus, and eczema erythematosus due to genetic, environmental, and immunological abnormalities and disruption of the skin barrier. Recently, the prevalence is increasing worldwide due to environmental changes, eating habits, and environmental pollution, and interest in solutions to these problems is also increasing.

The exact cause of atopic dermatitis is still unknown. However, Immunological characteristics associated with disease progression include a significant increase in the level of IgE antibody in the blood, and an increase in the expression level of interleukin-4 (IL-4), while a decrease in the expression of interferon gamma (IFNy)(Boguniewicz and Beltrani, 2002; AbdelHamid, 2003). Circulating IgE binds to two isoforms of IgE receptors. High-affinity IgE receptors (FcεRI) present on the surface of mast cells and basophiles, and low affinity IgE receptors (FcεRII or CD23) present on the surfaces of lymphocytes, eosinophils, platelets and macrophages. The onset of the symptoms of allergic disease is the cross-linking of IgE receptors on mast cells, and consequent degranulation of mast cells after meeting the allergen. The molecules released by mast cells include histamine, heparin, proteases and free radicals, which mediate a variety of biological effects including vasodilation, intestinal and/or bronchial smooth muscle contraction, mucous secretion and local proteolysis. Following initial immediate reaction of the mast cells, an influx of eosinophils, basophils and lymphocytes occurs 6 to 24 hours later. This late-stage response leads to chronic inflammation of tissues continuously exposed to antigens.

In general, drugs such as antihistamines, steroidal or non-steroidal anti-inflammatory drugs and leukotriene antagonists are used for the treatment of allergic diseases. Although these drugs are useful mainly for symptomatic effects, they do not provide the treatment required for the fundamental cure of allergic diseases, such as alleviating excessive humoral 1 immunity or suppressing IgE production. In addition, topical steroid preparations can cause various side effects, such as skin atrophy, vasodilation, loss of pigmentation, and development of dilated striatum (stretch marks) when used for a long period of time.

Accordingly, the present inventors confirmed that the newly synthesized pentapeptide can be usefully used for the prevention, improvement or treatment of allergic diseases including atopic dermatitis and completed the present.

[Disclosure]

[Technical Problem]

An object of the present invention is to provide a pharmaceutical composition for preventing or treating allergic diseases.

Another object of the present invention is to provide a cosmetic composition for preventing or improving allergic diseases.

Further another object of the present invention is to provide a composition for preventing or improving itching.

Further another object of the present invention is to provide a method for preventing or treating allergic diseases.

Further another object of the present invention is to provide a peptide for preventing or treating allergic diseases.

Further another object of the present invention is to provide a method for preventing or treating itching.

Further another object of the present invention is to provide a peptide for preventing or treating itching.

[Technical Solution]

In order to solve the above problems, one aspect of the present invention provides a pharmaceutical composition for the prevention or treatment of allergic diseases comprising a pentapeptide consisting of the amino acid sequence of SEQ ID NO: 1 (KFLIK) as an active ingredient.

Another aspect of the prevent invention provides a cosmetic composition for the prevention or improvement of allergic diseases comprising a pentapeptide consisting of the amino acid sequence of SEQ ID NO: 1 as an active ingredient.

Further another aspect of the prevent invention provides a method for the prevention or treatment of allergic diseases comprising administering a pentapeptide consisting of the amino acid sequence of SEQ ID NO: 1 to a subject.

Further another aspect of the prevent invention provides a pentapeptide consisting of the amino acid sequence of SEQ ID NO: 1 for the use of the prevention or treatment of allergic diseases.

Further another aspect of the prevent invention provides a composition for the prevention or improvement of itching comprising a pentapeptide consisting of the amino acid sequence of SEQ ID NO: 1 as an active ingredient.

Further another aspect of the prevent invention provides a method for the prevention or treatment of itching comprising administering a pentapeptide consisting of the amino acid sequence of SEQ ID NO: 1 to a subject.

Further another aspect of the prevent invention provides a pentapeptide consisting of the amino acid sequence of SEQ ID NO: 1 for the prevention or treatment of itching.

[Advantageous Effects]

The pentapeptide consisting of the amino acid sequence of SEQ ID NO: 1 of the present invention inhibits the release of β-hexosaminidase that induces itching and inflammatory responses, and significantly reduces the expression of inflammatory cytokines secreted due to the immune response of cells. Therefore, it can be used for preventing, improving or treating various allergic diseases including atopic dermatitis.

However, the effects of the present invention are not limited to the above-mentioned effects, and other effects not mentioned will be clearly understood by those skilled in the art from the following description.

[Description of Drawings]

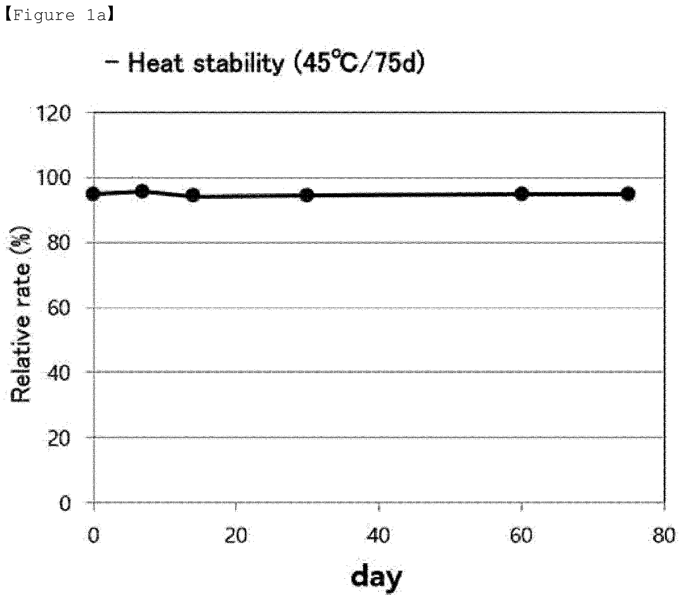

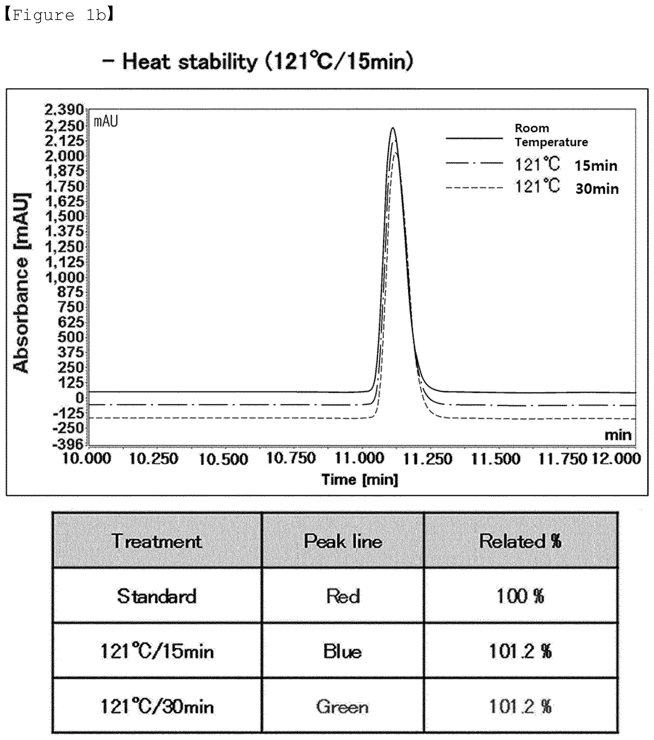

Fig. 1a and Fig. 1b show the results of the thermal stability evaluation of the KFLIK peptide:
Fig. 1a shows the results of confirming the high-temperature stability of the peptide during long-term storage at 45°C, and Fig. 1b shows the results of confirming the high-temperature stability of the peptide at the maximum heating temperature (121°C).
Fig. 2 is a graph showing the change in the amount of β-hexosamidase released by the KFLIK peptide treatment in mast cells.
Fig. 3 is a graph showing the expression change of inflammatory cytokines (IL-3, IL-4 and TNF-α) by the KFLIK peptide treatment in mast cells.
Fig. 4 is a graph showing the expression change of inflammatory cytokines (TNF-α, IL-1β and COX-2) by KFLIK peptide treatment in splenocytes.
Fig. 5a to Fig. 5c are graphs showing the improvement effect of atopic dermatitis by the KFLIK peptide of the present invention through human efficacy evaluation:

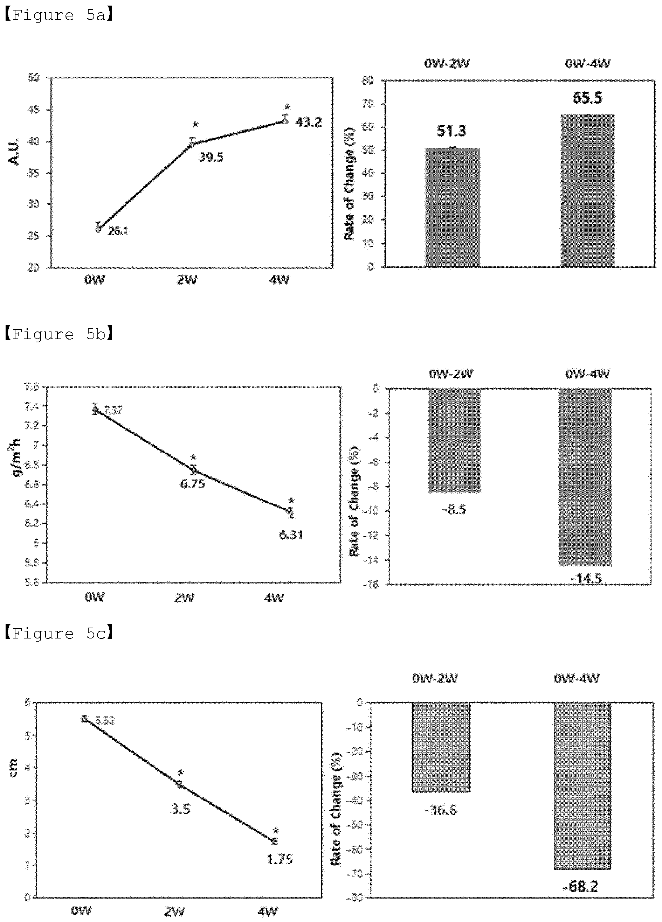

Fig. 5a shows the change rate (%) of skin moisture at the lesion site, Fig. 5b shows the change rate (%) of the amount of transdermal water loss at the lesion site, and Fig. 5c shows the change rate (%) of the skin itch index at the lesion site.

[Best Mode]

Hereinafter, the present invention will be described in detail.

One aspect of the present invention is to provide a pharmaceutical composition for the prevention or treatment of allergic diseases comprising a pentapeptide consisting of the amino acid sequence of SEQ ID NO: 1 as an active ingredient. Herein, the term 'peptide' refers to a linear or cyclic molecule formed by combining amino acid residues with each other by peptide bonds. The preparation of the peptide can be accomplished by conventional biological or chemical synthesis' methods known in the art, and for example, it can be accomplished by methods such as solid-phase synthesis techniques.

Herein, the term 'pentapeptide' refers to a linear molecule consisting of five amino acid residues, and the pentapeptide of the present invention refers to a linear peptide molecule composed of the amino acid sequence of SEQ ID NO: 1 (KFLIK).

The 'peptide' and 'pentapeptide' may be variants or fragments of amino acids having different sequences by deletion, insertion, substitution or a combination of amino acid residues within the range that does not affect the function. Amino acid exchanges that do not entirely alter the activity of the peptide are known in the art. In some cases, it may be modified by phosphorylation, sulfation, acrylation, glycosylation, methylation, farnesylation, etc. Accordingly, the present invention includes peptides having substantially the same amino acid sequence as the pentapeptide composed of the amino acid sequence of KFLIK, and variants or active fragments thereof. The substantially identical protein refers to an amino acid sequence having at least 75%, preferably at least 80%, for example, at least 85%, at least 90%, at least 95%, at least 98%, or at least 99% sequence homology with the amino acid sequence of KFLIK, but is not limited thereto, and it is included in the scope of the present invention if it has 75% or more amino acid sequence homology and has the same activity. In addition, the peptide of the present invention may further include a targeting sequence, a tag, a labeled residue, an amino acid sequence prepared for a specific purpose to increase the half-life or stability of the peptide.

In addition, in order to obtain better chemical stability, enhanced pharmacological properties (half-life, absorption, potency, efficacy, etc.), altered specificity (e.g., broad spectrum of biological activity), and reduced antigenicity, a protecting group may be bound to the N-terminus or C-terminus of the peptide of the present invention. For example, the protecting group may be acetyl group, fluorenyl methoxycarbonyl group, formyl group, palmitoyl group, myristyl group, stearyl group or polyethylene glycol (PEG), but if it is a component capable of modifying the peptide, particularly improving the stability of the peptide, it may be included without limitation. The 'stability' is used to include not only stability in vivo, which protects the peptide of the present invention from attack by proteolytic enzymes in vivo, but also storage stability (e.g., storage stability at room temperature).

The pentapeptide composed of the amino acid sequence of KFLIK can prevent or treat allergic diseases by inhibiting the expression of the inflammatory cytokines IL-3, IL-4, TNF-$\alpha$, IL-1$\beta$, and COX-2 secreted by the immune response.

Further, the pentapeptide composed of the amino acid sequence of KFLIK can prevent or treat allergic diseases by reducing the release of $\beta$-hexosaminidase that induces itching and inflammatory responses in mast cells.

Further, the pentapeptide composed of the amino acid sequence of KFLIK can improve or prevent the symptoms of atopic dermatitis by showing skin moisturizing, skin barrier improvement and itching improvement effects at the atopic dermatitis lesion site.

The 'allergic disease' refers to a disease caused by an allergic reaction in which the body's immune response to a foreign antigen is excessive, and may specifically be at least one disease selected from the group consisting of edema, anaphylaxis, allergic rhinitis, asthma, allergic conjunctivitis, allergic dermatitis, contact dermatitis, urticaria, pruritus, insect allergy, food allergy and drug allergy, but is not limited thereto.

The allergic dermatitis may be atopic dermatitis.

The 'atopic dermatitis' refers to a skin disease accompanied by symptoms such as itching, dry skin, increased skin thickness, and characteristic eczema as one of the allergic diseases.

The 'prevention' means any action that suppresses the onset or delays the onset by administration of the pentapeptide or the composition.

The 'treatment' or 'improvement' refers to any action in which the symptoms of the disease are improved or beneficially changed by the administration of the pentapeptide or the composition. The pharmaceutical composition for the prevention or treatment of allergic diseases according to the present invention can be formulated and used according to conventional methods in the form of oral formulations such as powders, granules, tablets, capsules, suspensions, emulsions, syrups and aerosols, external preparations, suppositories, and sterile injection solutions. For formulation, suitable carriers, excipients or diluents commonly used in the preparation of pharmaceutical compositions may be included.

The pharmaceutically acceptable carrier may include lactose, dextrose, sucrose, sorbitol, mannitol, starch, acacia, gum, calcium phosphate, alginate, gelatin, calcium silicate, microcrystalline cellulose, polyvinyl pyrrolidone, cellulose, water, syrup, methyl cellulose, methyl hydroxybenzoate, propyl hydroxybenzoate, talc, magnesium stearate, mineral oil and the like, commonly used in formulation.

The pharmaceutical composition may further include a lubricant, a wetting agent, a sweetening agent, a flavoring agent, an emulsifying agent, a suspending agent, a preservative, and the like, in addition to the above.

The pharmaceutical composition may be administered orally or parenterally (e.g., intramuscularly, intravenously, intraperitoneally, subcutaneously, intradermally, or topically) according to a desired method. The dosage varies depending on the condition and weight of the patient, the severity of the disease, the drug form, the route and time of administration, but may be appropriately selected by those skilled in the art.

The pharmaceutical composition of the present invention is administered in a pharmaceutically effective amount. In the present invention, a 'pharmaceutically effective amount' means an amount sufficient to treat a disease with a reasonable benefit/risk ratio applicable to medical treatment, and the effective dose level may be determined according to factors including the type and severity of the disease, drug activity, drug sensitivity, administration time, administration route and excretion rate, duration of treatment, and concurrent drugs and other well-known elements in the medical field. The pharmaceutical composition may be administered as an individual therapeutic agent or may be administered in combination with other therapeutic agents, may be administered simultaneously with, separately, or sequentially from a conventional therapeutic agent, and may be administered singly or multiple times. It is important to administer an amount that can obtain the maximum effect with a minimum amount without side effects, taking all of the above factors into consideration, and it can be easily determined by those skilled in the art.

The effective amount of the pharmaceutical composition may vary depending on the patient's age, sex, condition, body weight, absorption of the active ingredient into the body, inactivation rate, excretion rate, disease type, drug used in combination, and it can be increased or decreased depending on the route of administration, the severity of obesity, sex, weight, age, etc. For example, the pharmaceutical composition may be administered at about 0.0001 μg to 500 mg, preferably 0.01 μg to 100 mg per 1 kg of the patient's body weight per day.

In another aspect, the present invention provides a cosmetic composition for the improvement or relief of allergic diseases comprising a pentapeptide consisting of the amino acid sequence of SEQ ID NO: 1 as an active ingredient.

The specific details of the peptide, pentapeptide, allergic disease, and atopic dermatitis are as described above.

The cosmetic composition may be prepared in any formulation conventionally prepared in the art, and for example, it may be formulated in the form of solution, suspension, emulsion, paste, gel, cream, lotion, powder, soap, surfactant-containing cleansing, oil, powder foundation, emulsion foundation, wax foundation and spray, etc., but is not limited thereto.

The cosmetic composition may be prepared in various forms such as solution, sol gel, emulsion, oil, wax, aerosol, etc., for example, soft lotion, nourishing lotion, nourishing cream, massage cream, essence, eye cream, cleansing cream, cleansing foam, cleansing water, pack, spray, powder, hair tonic, hair cream, hair lotion, hair shampoo, hair rinse, hair conditioner, Hairspray, hair aerosol, pomade, gel and the like, but is not limited thereto.

The cosmetic composition of the present invention may include other additives such as excipients and carriers in addition to the pentapeptide composed of the amino acid sequence of KFLIK, and it is possible to apply and blend the usual ingredients that are combined to general cosmetics as needed.

When the formulation the cosmetic composition is paste, cream or gel, animal oil, vegetable oil, wax, paraffin, starch, tragacanth, cellulose derivative, polyethylene glycol, silicone, bentonite, silica, talc or zinc oxide, etc. may be used as a carrier ingredient.

When the formulation of the cosmetic composition is powder or spray, lactose, talc, silica, aluminum hydroxide, calcium silicate or polyamide powder may be used as a carrier ingredient. In particular, when the formulation is spray, a propellant such as chlorofluorohydrocarbon, propane/butane or dimethyl ether may be additionally included, but not limited thereto.

When the formulation of the cosmetic composition is solution or emulsion, a solvent, solubilizer or emulsifier may be used as a carrier component, for example, water, ethanol, isopropanol, ethyl carbonate, ethyl acetate, benzyl alcohol, benzyl benzoate, propylene glycol, 1,3-butyl glycol oil, glycerol aliphatic ester, polyethylene glycol or fatty acid ester of sorbitan may be used.

When the formulation of the cosmetic composition is suspension, a liquid diluent such as water, ethanol or propylene glycol, a suspending agent such as ethoxylated isostearyl alcohol, polyoxyethyl sorbitol ester and polyoxyethylene sorbitan ester, microcrystalline cellulose, aluminum metahydroxide, bentonite, agar or tragacanth and the like may be used as a carrier ingredient.

When the formulation of the cosmetic composition is surfactant-containing cleansing, aliphatic alcohol sulfate, aliphatic alcohol ether sulfate, sulfosuccinic acid monoester, isethionate, imidazolinium derivatives, methyl taurate, sarcosinate, fatty acid amide ether sulfate, alkylamidobetaine, fatty alcohol, fatty acid glyceride, fatty acid diethanolamide, vegetable oil, lanolin derivatives or ethoxylated glycerol fatty acid ester and the like can be used as a carrier ingredient.

When the formulation of the cosmetic composition is hair shampoo, the base ingredients for the composition of the shampoo, such as thickeners, surfactants, viscosity modifiers, moisturizers, pH adjusters, preservatives, essential oils, etc., may be mixed with the Trolox-peptide conjugate of the present invention. CDE may be used as the thickener, and LES, an anionic surfactant, and coco betaine, an amphoteric surfactant, may be used as the surfactant, Poly Quarter may be used as the viscosity modifier, glycerin may be used as the moisturizer, and citric acid and sodium hydroxide may be used as the pH adjuster. Grapefruit extract and the like may be used as the preservative, and in addition, essential oils such as cedarwood, peppermint, and rosemary, silk amino acids, pentaol, or vitamin E may be added.

The ingredients included in the cosmetic composition may further include ingredients commonly used in cosmetic compositions, for example, conventional adjuvants such as antioxidants, stabilizers, solubilizers, vitamins, pigments and fragrances, etc., in addition to the pentapeptide and carrier ingredients of the present invention as an active ingredient, but is not limited thereto.

In another aspect, the present invention provides a composition for the prevention or improvement of itching comprising a pentapeptide consisting of the amino acid sequence of SEQ ID NO: 1.

Further, the present invention provides a method for the prevention or treatment of itching comprising administering a pentapeptide consisting of the amino acid sequence of SEQ ID NO: 1 to a subject.

Further, the present invention provides a pentapeptide consisting of the amino acid sequence of SEQ ID NO: 1 for the prevention or treatment of itching.

The specific details of the peptide, pentapeptide, prevention, improvement are as described above.

The 'itching' refers to a skin condition that causes the unpleasant sensation of wanting to scratch, and it can be used in combination with 'pruritus'. The 'itching' includes itching by allergic diseases such as urticaria, psoriasis and atopic dermatitis, itching by ultraviolet rays, scalp itching, heat rash, eczema, frostbite, and pruritus in the elderly, but is not limited thereto.

Further, the present invention provides a method for the prevention or treatment of allergic diseases comprising administering a pentapeptide consisting of the amino acid sequence of SEQ ID NO: 1 to a subject.

The 'subject' may include a human. In addition, the term 'subject' may be a subject in need of administration of the pentapeptide of the present invention, and the subject in need of administration may include individuals who have been diagnosed with allergic disease or itching, individuals who have developed allergic diseases or itching-related symptoms, as well as individuals who wish to administer for the prevention of the disease or symptoms or health improvement.

The 'administration' means providing a predetermined substance to a patient by any suitable method, and the administration route of the composition of the present invention can be administered orally or parenterally through any general route as long as it can reach the target tissue. In addition, the composition may be administered by any device capable of transporting the active agent to a target cell.

The present invention provides a method for the prevention or treatment of allergic diseases pentapeptide consisting of the amino acid sequence of SEQ ID NO: 1.

As described above, the pentapeptide of the present invention inhibits mast cell degranulation to reduce the amount of $\beta$-hexosamidase released, and suppresses itching and inflammatory responses by inhibiting the expression of inflammatory cytokines. Therefore, it can be used as a raw material for a pharmaceutical or cosmetic composition for the purpose of preventing, improving or treating allergic diseases or itching.

Hereinafter, the present invention will be described in detail by way of Examples and Experimental Examples.

However, the following examples and experimental examples are only for illustrating the present invention, and the content of the present invention is not limited by the following examples and experimental examples.

[Example 1]

Synthesis and confirmation of physical properties of novel pentapeptide

1-1. Synthesis of pentapeptide

A novel peptide sequence 'KFLIK' consisting of the amino acid sequence of SEQ ID NO: 1 was prepared using a known method. As a result of measuring the molecular weight of the peptide containing the amino acid sequence of SEQ ID NO: 1 using a molecular weight measuring instrument, it was confirmed that the molecular weight corresponds to 647.4 Da.

1-2. Evaluation of high temperature stability during long-term storage

The peptide of the present invention consisting of the amino acid sequence of SEQ ID NO: 1 was dissolved in sterile distilled water at a concentration of 1000 ppm, stored at 45°C for 7 days, 14 days, 28 days, 60 days and 75 days, followed by HPLC analysis.

As a result, as shown in Fig. 1a, it was confirmed that the peptide of the present invention maintained stability for 75 days, which is the maximum observation day at 45°C.

1-3. Evaluation of high temperature stability

The peptide of the present invention was dissolved in sterile distilled water at a concentration of 1000 ppm, heated at 121°C for 15 minutes and 30 minutes, followed by HPLC analysis.

As a result, as shown in Fig. 1b, it was confirmed that the peptide of the present invention maintained stability for 30 minutes, which is the maximum heating time at 121°C.

**[Experimental Example 1]**

**Degranulation inhibitory effect by pentapeptide treatment in mast cells**

In order to confirm the effect of inhibiting allergic inflammatory response and improving atopic dermatitis by the pentapeptide of the present invention, the degree of degranulation was confirmed using mast cells (rat basophilic leukemia cell line, RBL-2H3).

Specifically, RBL-2H3 cells were inoculated into a 12-well plate at a density of $5 \times 10^5$ cells/well, and then cultured overnight at a 37°C, 5% $CO_2$ incubator. Then, the cells were sensitized by adding DNP-IgE to a complete medium. After 3 hours, it was washed 3 times with DPBS and then PIPES buffer was added thereto. In addition, an immune response was induced by pretreatment with the pentapeptide of the present invention synthesized in Example 1 for 30 minutes and then with DNP-HSA for 3 hours. After that, 25 μL of the supernatant was recovered, 25 μL of the substrate solution (5mM p-nitrophenyl-N-acetyl-β-D-glucosaminide dissolved in 0.2 M sodium citrate buffer, pH 4.5) was added thereto, and then incubated at 37°C for 90 minutes to react. After the reaction, 200 μL of stop solution (0.1 M $Na_2CO_3$/NaHCO_3$, pH 10.0) was added to each well to stop the enzymatic reaction, and absorbance was measured at 405 nm using a microplate reader. β-hexosamidase secreted from the supernatant was quantified according to Equation 1 below after adding 0.5% Triton X-100 solution to the cells.

[Equation 1]

% release= (experimental β-hexosaminidase release- spontaneous β-hexosaminidase release) / total cellular β-hexosaminidase x 100

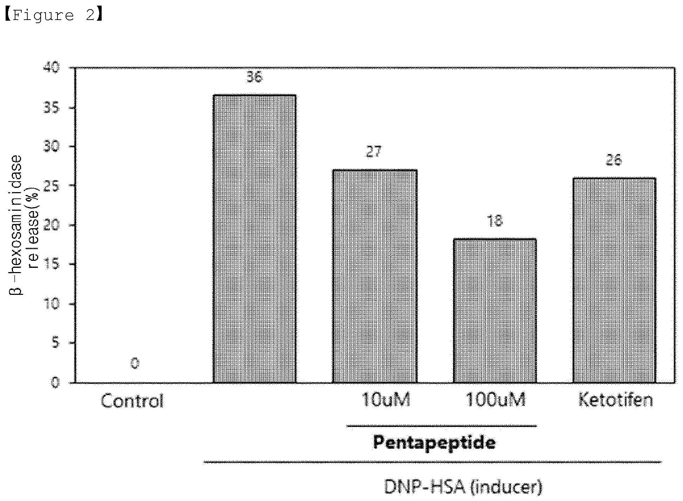

As a result, as shown in Fig. 2, in the control group (NC) sensitized with DNP-IgE and induced to an immune response with DNP-HSA, the release amount of β-hexosamidase was significantly increased compared to that of normal mast cells (NON). However, when 10 μM or 100 μM of the pentapeptide of the present invention was treated while sensitized with DNP-IgE and inducing an immune response with DNP-HSA, the release amount of β-hexosamidase was decreased in a concentration-dependent manner.

Through this, it can be seen that the pentapeptide of the present invention can effectively inhibit degranulation of mast cells and suppress itching and inflammatory reactions by reducing the amount of β-hexosamidase released from mast cells.

**[Experimental Example 2]**

**Effect of inhibition of expression of inflammation-related cytokines by pentapeptide treatment in mast cells**

In order to confirm the effect of inhibiting allergic inflammatory response and improving atopic dermatitis by the pentapeptide of the present invention, the expression pattern of inflammation-related cytokines was confirmed using mast cells (rat basophilic leukemia cell line, RBL-2H3).

Specifically, RBL-2H3 cells were inoculated into a 12-well plate at a density of $5 \times 10^5$ cells/well, and then cultured overnight at a 37°C, 5% $CO_2$ incubator. Then, the cells were sensitized by adding DNP-IgE to a complete medium. After 3 hours, it was washed 3 times with DPBS and then PIPES buffer was added thereto. In addition, an immune response was induced by pretreatment with the pentapeptide of the present invention synthesized in Example 1 for 30 minutes and then with DNP-HSA for 2 hours. At this time, 20 ug/mL of ketotifen was used as a positive control.

After recovering the cells and then isolating RNA, cDNA was synthesized from the isolated RNA using a cDNA synthesis kit (Intron, Korea). A polymerase chain reaction was performed using the synthesized cDNA, PCR premix (Intron, Korea) and IL-3, IL-4, and TNF-α primers, and the amplified PCR product was electrophoresed on an agarose gel to confirm a DNA band. The primer sequences used at this time are as shown in [Table 1].

[Table 1]

| Gene | Primer | Sequence (5'->3') | SEQ ID NO |
|---|---|---|---|
| IL-3 | F | CCA GAT TTC AGA CAG GGG CTC | 2 |
|  | R | CAG GTT TAC TCT CCG CAA GGT | 3 |
| IL-4 | F | TCC ACG GAT GTA ACG ACA GC | 4 |
|  | R | TCA TTC ACG GTG CAG CTT CT | 5 |
| TNF-α | F | ATG GGC TCC CTC TCA TCA GT | 6 |
|  | R | GAA ATG GCA AAT CGG CTG AC | 7 |

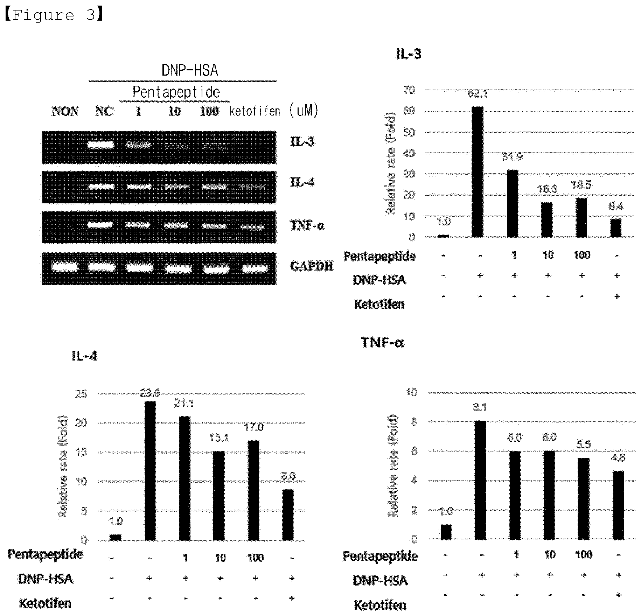

As a result, as shown in Fig. 3, in control cells (NC) sensitized with DNP-IgE and induced to an immune response with DNP-HSA, the expression of inflammatory cytokines IL-3, IL-4 and TNF-$\alpha$ was significantly increased compared to that of normal mast cells (NON). On the other hand, as a result of treatment with the pentapeptide of the present invention while sensitized with DNP-IgE and inducing an immune response with DNP-HSA, the expression of inflammatory cytokines increased by the immune response was significantly reduced. Through this, it can be seen that the pentapeptide of the present invention is effective in suppressing allergic inflammatory response and improving atopic dermatitis.

**[Experimental Example 3]**

**Effect of inhibition of expression of inflammation-related cytokines by pentapeptide treatment in splenocytes**

In order to confirm the effect of inhibiting allergic inflammatory response and improving atopic dermatitis by the pentapeptide of the present invention, the expression pattern of inflammation-related cytokines was confirmed using mouse splenocytes.

Specifically, after separating mouse splenocytes, the cells were inoculated into a 24-well plate at a density of $1\times10^7$ cells/well, and then cultured overnight at a 37°C, 5% $CO_2$ incubator. After changing the medium to a serum-free medium, 4 hours later, the pentapeptide of the present invention and TNF-$\alpha$ were treated and cultured for 30 minutes. At this time, 20 ug/mL of ketotifen was used as a positive control.

After recovering the cells and then isolating RNA, cDNA was synthesized using a cDNA synthesis kit (Intron, Korea). A polymerase chain reaction was performed using the synthesized cDNA, PCR premix (Intron, Korea) and TNF-$\alpha$, IL-1$\beta$ and COX-2 primers, and the amplified PCR product was electrophoresed on an agarose gel to confirm a DNA band. The primer sequences used at this time are as shown in [Table 2]..

[Table 2]

| Gene | Primer | Sequence(5'->3') | SEQ ID NO |
|---|---|---|---|
| TNF-$\alpha$ | F | AACATCCAACCTTCCCAAACG | 8 |
| | R | GACCCTAAGCCCCCAATTCTC | 9 |
| IL-1$\beta$ | F | TGAGTGGTAGCCAGCAAAGC | 10 |
| | R | CTGCAGTCCAGGTTCAATGG | 11 |
| COX-2 | F | TTCGACACATGGGATAACGA | 12 |
| | R | TCTTTCAACACGCAGGACAG | 13 |

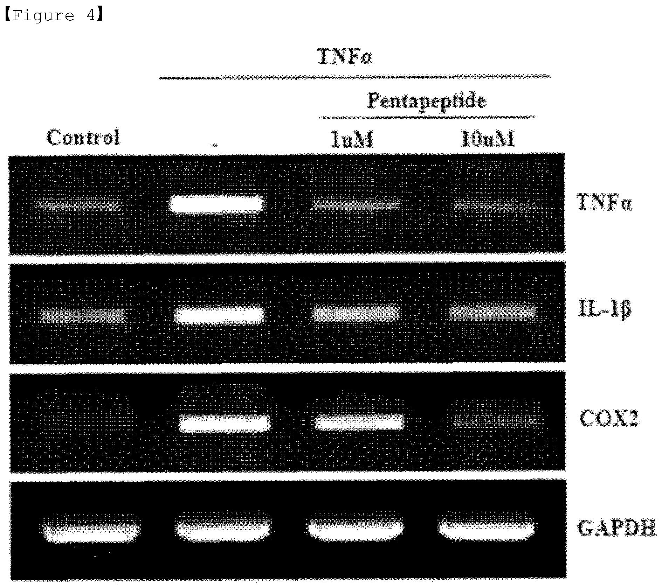

As a result, as shown in Fig. 4, in control cells (NC) induced to an immune response with TNF-$\alpha$, the expression of inflammatory cytokines TNF-$\alpha$, IL-1$\beta$ and COX-2 was significantly increased compared to that of normal mast cells (NON). On the other hand, as a result of treating splenocytes with the pentapeptide of the present invention together with TNF-$\alpha$, IL-1$\beta$ and COX-2, the expression of inflammatory cytokines increased by the immune response was significantly reduced. Through this, it can be seen that the pentapeptide of the present invention is effective in suppressing allergic inflammatory response and improving atopic dermatitis.

**[Experimental Example 4]**

**Effect of improving of atopic dermatitis through clinical trials**

Human efficacy evaluation was conducted for relieving itching caused by dryness in 21 patients with atopic. The dryness lesion site was used as the test site, and device measurement was performed on the affected area before using the product, 2 weeks after using the product, and 4 weeks after using the product.

The improvement effect of atopic dermatitis was confirmed by measuring skin moisture using Comeometer CM825, measuring skin moisture loss using Tewameter TM300, and evaluating improvement in itching (VAS).

4-1. Preparation of serum and cream containing pentapeptide

In order to confirm the improvement of atopic skin symptoms by the pentapeptide, serum and cream were prepared to contain 2000 ppm of the pentapeptide of the present invention synthesized in Example 1.

4-2. Skin moisturizing effect

As shown in Fig. 5a, it was confirmed that it showed statistically significant skin moisture improvement effect at the lesion area when the serum and cream containing the pentapeptide of the present invention were used (2 weeks, 4 weeks of use; $P < 0.05$).

4-3. Effect of improving skin barrier at lesion site

As shown in Fig. 5b, it showed statistically significant transdermal water loss improvement effect at the lesion area when the serum and cream containing the pentapeptide of the present invention were used [Change rate of transdermal water loss (%)=(after-before)/before*100].

4-4. Effect of improving itching

As shown in Fig. 5c, in the itching improvement effect, it was confirmed that the itching index was statistically significantly improved after 2 weeks and 4 weeks of use compared to before application when the serum and cream containing the pentapeptide of the present invention were used.

From this, it can be seen that the peptide serum and cream of the present invention exhibits skin moisturizing, skin barrier improvement and itching improvement effects, and can be used as a functional material for improving skin pruritus, moisture, and itching caused by skin dryness such as atopic dermatitis.

**Claims**

1.  A pharmaceutical composition for the prevention or treatment of allergic diseases comprising a pentapeptide consisting of the amino acid sequence of SEQ ID NO: 1 as an active ingredient.

2.  The pharmaceutical composition according to claim 1, wherein the allergic diseases include at least one selected form the group consisting of edema, anaphylaxis, allergic rhinitis, asthma, allergic conjunctivitis, allergic dermatitis, contact dermatitis, urticaria, itching, insect allergy, food allergy and drug allergy.

3.  The pharmaceutical composition according to claim 2, wherein the allergic dermatitis is atopic dermatitis.

4.  The pharmaceutical composition according to claim 1, wherein the pentapeptide is i) inhibiting the expression of IL-4, IL-13, TNF-$\alpha$, IL-1$\beta$, IL-6 or IL-8, and ii) inhibiting the release of $\beta$-hexosaminidase.

5.  The pharmaceutical composition according to claim 1, which further comprises a pharmaceutically acceptable carrier, an excipient or a diluent.

6.  The pharmaceutical composition according to claim 1, which is formulated in the form of oral preparation, injection preparation or external preparation.

7.  The pharmaceutical composition according to claim 5, wherein the external preparation is any one or more selected from the group consisting of creams, gels, ointments, emulsions, suspensions, sprays and transdermal delivery patches.

8.  A cosmetic composition for the prevention or improvement of allergic diseases comprising a pentapeptide consisting of the amino acid sequence of SEQ ID NO: 1 as an active ingredient.

9.  The cosmetic composition according to claim 8, wherein the allergic diseases include at least one selected form the group consisting of allergic dermatitis, contact dermatitis, urticaria, itching, psoriasis and eczema..

10. The cosmetic composition according to claim 8, wherein the allergic dermatitis is atopic dermatitis.

11. The cosmetic composition according to claim 8, which further comprises a cosmetically acceptable carrier, an excipient or a diluent.

12. The cosmetic composition according to claim 8, which is prepared in any one or more formulations selected from the group consisting of skin, lotion, cream, essence, emulsion, gel, hand cream, lipstick, cleansing foam, cleansing cream, cleansing water, spray, shampoo, conditioner, treatment, body cleanser, soap, pack, massage agent, face powder, compact, foundation, two-way cake, and makeup base.

**13.** A composition for the prevention or improvement of itching comprising a pentapeptide consisting of the amino acid sequence of SEQ ID NO: 1 as an active ingredient.

【Figure 1a】

Heat stability (45°C/75d)

【Figure 1b】

## – Heat stability (121℃/15min)

| Treatment | Peak line | Related % |
|---|---|---|
| Standard | Red | 100 % |
| 121℃/15min | Blue | 101.2 % |
| 121℃/30min | Green | 101.2 % |

【Figure 2】

【Figure 3】

【Figure 4】

【Figure 5a】

【Figure 5b】

【Figure 5c】

## INTERNATIONAL SEARCH REPORT

| | International application No. |
|---|---|
| | **PCT/KR2021/007322** |

### A. CLASSIFICATION OF SUBJECT MATTER

**A61K 38/08**(2006.01)i; **A61P 37/08**(2006.01)i; **A61P 11/06**(2006.01)i; **A61P 27/14**(2006.01)i; **A61P 17/00**(2006.01)i; **A61K 8/64**(2006.01)i; **A61Q 19/00**(2006.01)i

According to International Patent Classification (IPC) or to both national classification and IPC

### B. FIELDS SEARCHED

Minimum documentation searched (classification system followed by classification symbols)

A61K 38/08(2006.01); A61K 38/17(2006.01); A61K 45/06(2006.01); A61K 8/02(2006.01); A61L 2/16(2006.01); C07K 14/00(2006.01); C07K 14/47(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: KFLIK, 펜타펩타이드(pentapeptide), 알러지(allergy), 피부염(dermatitis), 아토피 (atopy), 가려움증(itching)

### C. DOCUMENTS CONSIDERED TO BE RELEVANT

| Category* | Citation of document, with indication, where appropriate, of the relevant passages | Relevant to claim No. |
|---|---|---|
| A | US 2016-0279193 A1 (NORWEGIAN UNIVERSITY OF SCIENCE AND TECHNOLOGY (NTNU)) 29 September 2016 (2016-09-29)<br>See abstract; SEQ ID NO: 269; and claim 1. | 1-13 |
| A | US 2016-0289272 A1 (NORWEGIAN UNIVERSITY OF SCIENCE AND TECHNOLOGY) 06 October 2016 (2016-10-06)<br>See abstract; SEQ ID NO: 269; and claim 1. | 1-13 |
| A | KR 10-2020-0020623 A (FELIZCOS CO., LTD. et al.) 26 February 2020 (2020-02-26)<br>See abstract; and claims 1-17. | 1-13 |
| A | KR 10-2017-0114921 A (INDUSTRY-ACADEMIC COOPERATION FOUNDATION, YONSEI UNIVERSITY) 16 October 2017 (2017-10-16)<br>See abstract; and claims 1-15. | 1-13 |

☑ Further documents are listed in the continuation of Box C.    ☑ See patent family annex.

| | |
|---|---|
| *   Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **09 September 2021** | **09 September 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
|---|
| **PCT/KR2021/007322** |

### C. DOCUMENTS CONSIDERED TO BE RELEVANT

| Category* | Citation of document, with indication, where appropriate, of the relevant passages | Relevant to claim No. |
|---|---|---|
| A | KR 10-2017-0018485 A (APIM THERAPEUTICS AS) 17 February 2017 (2017-02-17) See abstract; and claims 1-24. | 1-13 |

Form PCT/ISA/210 (second sheet) (July 2019)

**INTERNATIONAL SEARCH REPORT**

International application No.

**PCT/KR2021/007322**

**Box No. I       Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

         ☑ in the form of an Annex C/ST.25 text file.

         ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

         ☐ in the form of an Annex C/ST.25 text file (Rule 13*ter*.1(a)).

         ☐ on paper or in the form of an image file (Rule 13*ter*.1(b) and Administrative Instructions, Section 713).

2.  ☐   In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

**INTERNATIONAL SEARCH REPORT**
Information on patent family members

International application No.

**PCT/KR2021/007322**

| Patent document cited in search report | | | Publication date (day/month/year) | Patent family member(s) | | | Publication date (day/month/year) |
|---|---|---|---|---|---|---|---|
| US | 2016-0279193 | A1 | 29 September 2016 | DK | 3065759 | T3 | 17 May 2021 |
| | | | | EP | 3065759 | A1 | 14 September 2016 |
| | | | | EP | 3065759 | B1 | 31 March 2021 |
| | | | | PT | 3065759 | T | 17 May 2021 |
| | | | | US | 10517923 | B2 | 31 December 2019 |
| | | | | US | 2020-0323953 | A1 | 15 October 2020 |
| | | | | WO | 2015-067712 | A1 | 14 May 2015 |
| US | 2016-0289272 | A1 | 06 October 2016 | CA | 2929454 | A1 | 14 May 2015 |
| | | | | EP | 3065760 | A1 | 14 September 2016 |
| | | | | EP | 3065760 | B1 | 08 July 2020 |
| | | | | ES | 2824273 | T3 | 11 May 2021 |
| | | | | US | 10450348 | B2 | 22 October 2019 |
| | | | | US | 2020-0095286 | A1 | 26 March 2020 |
| | | | | WO | 2015-067713 | A1 | 14 May 2015 |
| KR | 10-2020-0020623 | A | 26 February 2020 | KR | 10-2281774 | B1 | 27 July 2021 |
| KR | 10-2017-0114921 | A | 16 October 2017 | KR | 10-2019-0111870 | A | 02 October 2019 |
| | | | | KR | 10-2028245 | B1 | 04 October 2019 |
| | | | | KR | 10-2106466 | B1 | 28 May 2020 |
| KR | 10-2017-0018485 | A | 17 February 2017 | AU | 2009-216567 | A1 | 27 August 2009 |
| | | | | AU | 2009-216567 | B2 | 07 November 2013 |
| | | | | BR | PI0908853 | A2 | 30 May 2017 |
| | | | | BR | PI0908853 | B1 | 25 May 2021 |
| | | | | CA | 2715940 | A1 | 27 August 2009 |
| | | | | CA | 2715940 | C | 30 June 2020 |
| | | | | CN | 102015758 | A | 13 April 2011 |
| | | | | CN | 102015758 | B | 12 April 2017 |
| | | | | DK | 2254904 | T3 | 06 June 2018 |
| | | | | EP | 2254904 | A2 | 01 December 2010 |
| | | | | EP | 2254904 | B1 | 11 April 2018 |
| | | | | EP | 3338790 | A2 | 27 June 2018 |
| | | | | EP | 3338790 | A3 | 11 July 2018 |
| | | | | ES | 2670334 | T3 | 30 May 2018 |
| | | | | HR | P20180718 | T1 | 13 July 2018 |
| | | | | HU | E037737 | T2 | 28 September 2018 |
| | | | | IL | 207384 | B | 31 May 2018 |
| | | | | JP | 2011-512150 | A | 21 April 2011 |
| | | | | JP | 2015-180634 | A | 15 October 2015 |
| | | | | JP | 5863240 | B2 | 16 February 2016 |
| | | | | JP | 6116614 | B2 | 19 April 2017 |
| | | | | KR | 10-1653774 | B1 | 05 September 2016 |
| | | | | KR | 10-1749204 | B1 | 20 June 2017 |
| | | | | KR | 10-2010-0123728 | A | 24 November 2010 |
| | | | | KR | 10-2016-0038903 | A | 07 April 2016 |
| | | | | LT | 2254904 | T | 11 June 2018 |
| | | | | MX | 2010008989 | A | 07 December 2010 |
| | | | | NO | 2254904 | T3 | 08 September 2018 |
| | | | | PL | 2254904 | T3 | 31 August 2018 |
| | | | | PT | 2254904 | T | 04 June 2018 |
| | | | | RU | 2010134786 | A | 27 March 2012 |
| | | | | RU | 2549675 | C2 | 27 April 2015 |

Form PCT/ISA/210 (patent family annex) (July 2019)

**INTERNATIONAL SEARCH REPORT**
Information on patent family members

International application No.

**PCT/KR2021/007322**

| Patent document cited in search report | Publication date (day/month/year) | Patent family member(s) | | | Publication date (day/month/year) |
|---|---|---|---|---|---|
| | | TR | 201807218 | T4 | 21 June 2018 |
| | | US | 10213483 | B2 | 26 February 2019 |
| | | US | 2011-0020437 | A1 | 27 January 2011 |
| | | US | 2015-0017232 | A1 | 15 January 2015 |
| | | US | 2017-0246248 | A1 | 31 August 2017 |
| | | US | 8871724 | B2 | 28 October 2014 |
| | | US | 9676822 | B2 | 13 June 2017 |
| | | WO | 2009-104001 | A2 | 27 August 2009 |
| | | WO | 2009-104001 | A3 | 15 April 2010 |
| | | ZA | 201005506 | B | 26 October 2011 |
| | | ZA | 201105685 | B | 25 May 2012 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AMIN K.** *Respiratory Medicine.*, 2012